# EUROPÄISCHE PATENTANMELDUNG

(11) **EP 1 167 538 A1**
(43) Veröffentlichungstag der Anmeldung: **02.01.2002**
(21) Anmeldenummer: 00113906.2
(22) Anmeldetag: 30.06.2000
(51) Int. Cl.: C12Q 1/00, G01N 27/30

(54) **Biosensor und Herstellverfahren dafür**

(71) Anmelder: Schibli Engineering GmbH, 4503 Solothurn (CH)
(72) Erfinder: Schibli, Peter Urs, 4503 Solothurn (CH)
(74) Vertreter: Störle, Christian, Dr.

(57) **Zusammenfassung**

Die Anmeldung betrifft einen Biosensor zum Bestimmen von Substanzen in Körperflüssigkeiten, insbesondere in Blut, mit einer zweiteiligen Trägerplatte, wobei das eine Teil der Trägerplatte ein Oberteil und das andere Teil ein Unterteil darstellt, und einer zwischen Oberteil und Unterteil liegenden Zwischenlage in der ein Schlitz gebildet ist, wobei Oberteil, Unterteil und Schlitz einen Kapillarkanal bilden, der von einer am Rand des Biosensors gebildeten Auftragsöffnung zu einem im Ober- oder Unterteil gebildeten Luftloch verläuft, und Elektroden vorgesehen sind, die zusammen mit einer enzymhaltigen Substanz eine elektrochemische Messung von in Körperflüssigkeiten befindlichen Substanzen erlauben, wobei das Oberteil und das Unterteil im Bereich des Kapillarkanals mindestens je eine Elektrode trägt, die paarweise gegenüberliegen und so angeordnet sind, daß beide Elektrodenpaare im Kapillarkanal liegende Meßbereiche bilden, wobei auf mindestens einer Elektrode jedes Elektrodenpaars eine enzymhaltige Substanz aufgebracht ist.

## Beschreibung

Die Erfindung bezieht sich auf einen Biosensor zum Bestimmen von Substanzen in Körperflüssigkeiten sowie ein Herstellverfahren für einen solchen Biosensor.

Die Bestimmung von Substanzen in Körperflüssigkeiten mittels Biosensoren ist bekannt. Beispielsweise kann man Glucose im Harn oder im Blut mittels des Enzmys Glucoseoxidase bestimmen (vgl. Carlson, "Kurzes Lehrbuch der Biochemie für Mediziner und Naturwissenschaftler", 11. Auflage, S. 189). Zur Durchführung wird z. B. die in einem Bluttropfen enthaltene Glucose durch Glucoseoxidase zu Gluconolacton oxidiert. Die dabei freiwerdenden Elektronen reduzieren das Enzym Glucoseoxidase, welches wiederum Elektronen auf einen Mediator, z. B. Ferrocen, überträgt, wobei das Enzym seinerseits oxidiert wird. Vom Mediator können die Elektronen auf eine Elektrode übertragen werden, so daß bei Anlegen einer Spannung ein Mikrostrom fließt. Dieser Strom kann als Maß für den Glucosegehalt der Blutprobe verwendet werden. Diese enzymatische Reaktion wird in einem Biosensor also elektrochemisch erfaßt, indem nach Anlegen einer Spannung ein Strom zwischen zwei Elektroden gemessen wird. Nach diesem Prinzip arbeitende Biosensoren sind beispielsweise aus der US-PS 5,264,130, der US-PS 5,264,106 oder der EP-A 0 359 831 bekannt.

Letzere Druckschrift, von der im Oberbegriff des Anspruchs 1 ausgegangen wurde, zeigt einen aus einer zweiteiligen Trägerplatte aufgebauten Biosensor, wobei ein Teil das Oberteil und das andere Teil das Unterteil darstellt, und zwischen Ober- und Unterteil eine Zwischenlage vorgesehen ist, in der sich ein Schlitz befindet. Dieser Schlitz endet einerseits in einem Luftloch und andererseits in einer Öffnung im Rande des Biosensors, an der Körperflüssigkeit zugeführt wird. Auf dem Unterteil ist im Bereich des Schlitzes eine Elektrodenanordnung und eine enzymhaltige Substanz vorgesehen, mit der die erwähnte elektrochemische Messung von Substanzen möglich ist.

Der Erfindung liegt die Aufgabe zugrunde, einen gattungsgemäßen Biosensor hinsichtlich seiner Nachweiseigenschaften zu verbessern und ein Herstellverfahren für einen solchen Biosensor anzugeben.

Erfindungsgemäß wird bei einem Biosensor zum Bestimmen von Substanzen in Körperflüssigkeiten mit einer zweiteiligen Trägerplatte, wobei das eine Teil der Trägerplatte ein Oberteil und das andere Teil ein Unterteil darstellt, und einer zwischen Oberteil und Unterteil liegenden Zwischenlage in der ein Schlitz gebildet ist, wobei Oberteil, Unterteil und Schlitz einen Kapillarkanal bilden, der von einer am Rand des Biosensors gebildeten Auftragsöffnung zu einem im Ober- oder Unterteil gebildeten Luftloch verläuft, und Elektroden vorgesehen sind, die zusammen mit einer enzymhaltigen Substanz eine elektrochemische Messung von in Körperflüssigkeiten befindlichen Substanzen erlauben, diese Aufgabe dadurch gelöst, daß das Oberteil und das Unterteil im Bereich des Kapillarkanals mindestens jeweils eine Elektrode tragen, wobei die Elektroden paarweise gegenüberliegen und so angeordnet sind, daß jedes Elektrodenpaar einen im Kapillarkanal liegenden Meßbereich bildet, wobei auf mindestens einer Elektrode mindestens eines Elektrodenpaars eine enzymhaltige Substanz aufgebracht ist,

Wesentlich für dieses erfindungsgemäße Konzept ist, daß die Elektroden nicht ausschließlich auf dem Unterteil eines mehrteiligen Sensor liegen. Statt dessen wird die auf dem Oberteil zur Verfügung stehende Fläche erfindungsgemäß ebenfalls mit Elektroden bestückt, so daß die Elektroden paarweise gegenüberliegen.

Dieser Biosensor hat den Vorteil, daß durch die paarweise gegenüberliegenden Elektroden die wirksame Elektrodenfläche gegenüber dem Stand der Technik stark vergrößert ist. Dadurch steigt das direkt von der Elektrodenfläche abhängende Nutzsignal bei einer elektrochemischen Messung. Somit ist das Signal/Rausch-Verhältnis deutlich verbessert, wodurch die mit dem Biosensor erreichbaren Nachweisgrenzen für die zu erfassenden Substanzen sinkt. Das verbesserte Signal/Rausch-Verhältnis macht es möglich, Analysen mit einer geringeren Körperflüssigkeitsmenge durchzuführen, beispielsweise ist die für eine Blutzuckermessung nötige Mindestblutmenge deutlich geringer, was für den Patienten angenehmer, weil weniger schmerzempfindlich bei Abnahme eines Bluttropfens ist.

Die Elektroden paarweise gegenüber liegend anzuordnen, hat weiter den Vorteil, daß nahezu beliebig viele Elektrodenpaare im Kapillarkanal hintereinander liegend vorgesehen werden können. Dies ermöglicht es nicht nur eine, sondern gleich mehrere Substanzen in einer Körperflüssigkeitsmeßprobe nachzuweisen.

Da die Elektroden paarweise gegenüberliegen, ergibt sich weiter der Vorteil, daß die zu analysierende Körperflüssigkeit genau zwischen allen Elektrodenpaaren durchströmt und nicht nacheinander über paarweise zugeordnete hintereinanderliegende Elektroden hinwegläuft. Der von den Elektroden aufgenommene Strom fließt also quer zur Fließrichtung der Körperflüssigkeit, was unter Gesichtspunkten der Meßgenauigkeit vorteilhaft ist, da das Meßsignal mit dem Eintreten der Körperflüssigkeit in den Zwischenraum zwischen jedem Elektrodenpaar sprungartiger ansteigt, als wenn die Flüssigkeit über hintereinanderliegende Elektroden fließen würde. Somit ist die für eine Analyse nötige Mindestmenge an Körperflüssigkeit weiter abgesenkt.

Besondere fertigungstechnische Vorteile ergeben sich, wenn eine Trägerplatte verwendet wird, die entlang einer Knicklinie zusammengeklappt ist, d. h. wenn das Ober- und Unterteil Teile einer einstückigen Trägerplatte sind. Dies vereinfacht Aufbringen und Kontaktieren der Elektroden, da es vor dem Zusammenklappen in einem Arbeitsschritt und auf eine Fläche eines Bauteiles erfolgen kann. Darüber hinaus wird durch das Zusammenklappen der Trägerplatte entlang einer Ober- und Unterteil trennenden Knicklinie eine besonders gute Passung der paarweise gegenüberliegenden Elektroden sichergestellt, wodurch Paß- und Justierstrukturen unnötig sind. Auch ist das Aufbringen der enzymhaltigen Substanz auf der Trägerplatte vor dem Zusammenklappen einfacher. Dieses Konzept ermöglicht es weiter, die den Schlitz aufweisende Zwischenlage relativ einfach, beispielsweise durch ein Siebdruckverfahren, auf die Trägerplatte aufzubringen.

In dieser Weiterbildung kann man dann, da die Elektroden in einem Arbeitsschritt auf die Trägerplatte vor dem Zusammenklappen aufbringbar sind, alle Elektroden über Kontakte kontaktieren, die entweder auf dem Ober- oder auf dem Unterteil liegen. Vorzugsweise ist dazu ein im zusammengeklappten Zustand überstehender die Kontakte tragender Abschnitt am Ober- oder Unterteil vorgesehen, der zum Einstecken in ein Auswertegerät ausgebildet ist, das dann über die Kontakte eine elektrische Verbindung zu den Elektroden herstellt.

Für das Material für Ober- und Unterteil bzw. die Trägerplatte kommt jede Substanz in Frage, die zum einen ausreichend inert bezüglich der zu analysierenden Körperflüssigkeit und der Enzympaste ist, um Querempfindlichkeiten und Meßfehler zu vermeiden, und die zum anderen durch ihre Benetzungseigenschaften eine Kapillarwirkung im Kapillarkanal ermöglicht. Besonders bevorzugt sind dünne Folien, wobei Kapton oder Polyester aus Kostengesichtspunkten vorteilhaft ist. Zur Versteifung kann man eine stabilisierende Unterschicht unter der Folie des Unterteils vorsehen; dann können die Folien besonders dünn gewählt werden.

Die Auftragsöffnung zum Einbringen der zu analysierenden Körperflüssigkeit in den Kapillarkanal kann prinzipiell überall liegen. Er muß bloß bis an einen Rand des Biosensors reichen, beispielsweise bis an den Rand der Trägerplatte. Besonders bevorzugt ist es jedoch, wenn im Bereich der Knicklinie ein Loch in der Trägerplatte vorgesehen wird, bis zu dem der Schlitz in der Zwischenlage reicht. Dieses Loch bildet dann nach dem Zusammenklappen der Trägerplatte die Auftragsöffnung. Besonders bequem, beispielsweise für einen Patienten, der aus einem Finger einen Bluttropfen in die Auftragsöffnung einbringen möchte, ist ein Biosensor, wenn die am von der Knicklinie gebildeten Rand vorgesehene Auftragsöffnung eine bogenförmige Einbuchtung, beispielsweise mit dem Profil einer Fingerkuppe, hat. Dann muß der Patient seinen Finger nach dem zum Gewinnen eines Bluttropfens üblichen Stechen mit einer Nadel lediglich in die Einbuchtung am Rand des Biosensors legen.

Beim Aufbringen eines solchen Bluttropfens von einer Fingerbeere in eine Auftragsöffnung stellt es sich für den Patienten regelmäßig als problematisch dar, zu wissen, ob er seinen Bluttropfen auch in die Auftragsöffnung hineingebracht hat. Dieses Problem stellt sich dem Patienten nicht mehr, wenn gemäß einer bevorzugten Weiterbildung des erfindungsgemäßen Biosensors die Einbuchtung am Rand des Biosensors im Unterteil bezogen auf die Deckfläche des Biosensors eine senkrecht liegende und im Oberteil bezogen auf die Deckfläche eine vorspringende, insbesondere eine schräg liegende Randfläche hat. Besonders bevorzugt ist es dabei, daß die schräg liegende Randfläche mit ihrer stumpfen Kante mit der senkrechten Randfläche der Einbuchtung im Unterteil weitgehend fluchtet. Eine derart gestaltete vorstehende Schrägfläche wirkt quasi als Verdrehsicherung. Bei der Bauweise mit der Trägerplatte kann man das Loch dann ganz bewußt asymmetrisch gestalten, so daß der im Unterteil liegende Rand des Loches nach dem Zusammenklappen nicht genau über dem Oberteil in dem Rand des Loches zu liegen kommt.

Mit dieser Verdrehsicherung muß ein Patient den Finger dann nicht mehr auf eine schmale senkrecht zum flachen Biosensor liegenden Randfläche auflegen, sondern kann aufgrund des vorstehenden, insbesondere schrägen Randes der Einbuchtung am Oberteil mitverfolgen, ob er mit dem Bluttropfen auch die Eintrittsöffnung in den Kapillarkanal trifft. Diese Verdrehsicherung ist besonders wirksam, wenn die schräg verlaufende Fläche unter einen Winkel von etwa 30 bis 40° zur Deckfläche verläuft.

Dieses Konzept ist insbesondere vor dem Hintergrund von großem Vorteil, daß auch ältere Patienten solche Biosensoren verwenden.

Die Ausbildung des Kapillarkanals kann durch die Formung des Schlitzes nahezu beliebig gestaltet werden, so lange dafür Sorge getragen ist, daß eine Kapillarwirkung besteht, d. h. daß an der Aufnahmeöffnung eingebrachte Körperflüssigkeit auch durch Kapillarkräfte entlang des Kapillarkanals transportiert wird. In einer Weiterbildung der Erfindung wird durch die Gestaltung des Kapillarkanals als Schrägkapillarkanal die Transportgeschwindigkeit der Körperflüssigkeit im Kanal gesteuert. Gestaltet man den Kapillarkanal aufweitend, d. h. nimmt die Breite des Schlitzes in der Zwischenlage und damit die Breite des Kapillarkanals von der Auftragsöffnung weg gesehen zu, so wird die Körperflüssigkeit von der Auftragsöffnung schnell weggefördert. Gestaltet man die Breite des Schlitzes und mithin den Querschnitt des Kapillarkanals abnehmend, erfolgt ein langsames Absaugen. Somit kann man die Ansprechgeschwindigkeit des Biosensors anwendungsgemäß gestalten.

Die Zwischenlage hat die Funktion zusammen mit dem Ober- und dem Unterteil den Kapillarkanal zu bilden. Sie ist deshalb wie das Material für Ober- und Unterteil gegenüber der zu analysierenden Körperflüssigkeit inert und so beschaffen, daß sie nicht zu Meßfehlern führt. Weiter schädigt sie die verwendeten Enzyme nicht.

Besonders bevorzugt ist aus Fertigungsgründen eine zweilagige Zwischenlage, die aus einer auf das Unterteil aufgebrachten Lackschicht von geeigneter Dicke besteht, die über eine Klebemasse mit dem Oberteil verklebt ist. Die Klebemasse ist so beschaffen, daß ihr Aushärtevorgang die Enzyme nicht schädigt. Dies kann insbesondere durch feuchtigkeitsabbindende oder druckaktivierbare Klebstoffe erreicht werden.

Die Kommunikation mit dem den Biosensor anwendenden Patienten ist wichtig. In einer Weiterbildung des Biosensors befindet sich deshalb an beiden Rändern des den überstehenden Abschnitts aufweisenden Ober- oder Unterteils ein Lichtleitelement, das nach Einstecken in das Auswertegerät beleuchtet werden kann. Beispielsweise leuchtet je nach Einstrahlung durch das Auswertegerät dieses Lichtleitelements in einer bestimmten Farbe, z. B. in rot oder in grün. Dieses Lichtleitelement kann durch eine Druckprägung des Randes des Ober- und/oder Unterteils erreicht werden, so daß sich ein das Licht leitender Strahlungskanal als Form einer Leitlinie am Sensorrand ergibt. Im Auswertegerät befindet sich dann eine rote bzw. eine grüne Leuchtquelle, beispielsweise eine LED. Je nach von ihr eingestrahltem Licht, weis der Patient nun, was zu tun ist.

Für die enzymhaltige Substanz sind prinzipiell alle Enzyme tauglich, die eine elektrochemische Messung ermöglichen. Besonders bevorzugt ist es, das Enzym aus folgender Gruppe auszuwählen: Lactatoxidase, Glucoseoxidase, Cholesterase, Uricase, Xanthinoxidase, Peroxidase, Urease, Aminotransferase, Cholesterinoxidase, Aminooxidase, Glutamatoxidase, Kreatininoxidase, Kreatininaminohydrolase und Dehydrogenasen. Natürlich kann man bei einzelnen Elektrodenpaaren unterschiedliche enzymhaltige Substanzen verwenden. Damit kann der Biosensor an einer Körperflüssigkeitsprobe mehrere verschiedene Substanzen messen. Natürlich ist es auch möglich, ein Elektrodenpaar als Referenzelektrodenpaar zu verwenden, um einen Nullwert zu gewinnen, wie es nach dem Stand der Technik bekannt ist

Die Herstellung des erfindungsgemäßen Biosensors erfolgt durch:
a) Herstellen einer Trägerplatte, die zwei Teile aufweist, welche um eine Knicklinie zusammenklappbar sind, wobei das eine Teil der Trägerplatte ein Oberteil und das andere Teil ein Unterteil des Biosensors darstellt,
b) Bilden eines Durchtrittsloches in der Trägerplatte,
c) Aufbringen einer Leiterstruktur aufweisend Elektroden, die durch Leiterbahnen mit Kontakten auf dem Ober- oder dem Unterteil verbunden sind, wobei zwei Elektroden auf dem Oberteil und zwei Elektroden auf dem Unterteil sitzen, und
d) Aufbringen einer Zwischenlage auf das Ober- oder das Unterteil, wobei in der Zwischenlage ein Schlitz vorgesehen wird, der zum Loch läuft und über den Elektroden des Ober- oder Unterteils liegt.

Dieses Verfahren ermöglicht es, die Leiterstruktur und die Zwischenlage mit Siebdruckverfahren herzustellen. Siebdruckverfahren sind zum einen sehr kostengünstig, zum anderen erlauben sie eine sehr exakte Positionierung der Elektroden bzw. der Strukturen der Zwischenlage. Bei der Sensorvariante, die entlang der Knicklinie zusammengeklappt wird, ist durch den Klappvorgang zugleich eine exakte Passung der gegenüberliegenden Elektrodenpaare sichergestellt.

Bei Biosensoren stellt sich regelmäßig das Problem, daß die enzymhaltigen Substanzen nach der Zubereitung nur relativ kurz gelagert werden dürfen. Darüber hinaus sind oft spezielle Lagerbedingungen, z. B. niedrige Temperatur, einzuhalten. Das erfindungsgemäße Herstellverfahren ermöglicht es, die Herstellung des Biosensors nach dem Aufbringen der Zwischenlage zu unterbrechen. Wählt man als Zwischenlage eine Klebschicht, kann diese mit einem Schutzblatt abgedeckt werden. Bis dahin kann der Biosensor in sehr großen Stückzahlen gefertigt und beliebig lange gelagert werden. Um dann die für den Verbrauch in nächster Zukunft vorgesehene Menge an Biosensoren fertigzustellen, muß man nur noch das Schutzblatt abziehen und die enzymhaltige Substanz aufbringen. Verwendet man die Bauform mit zweilagiger Zwischenlage, so kann bei Einsatz eines druckaktivierbaren Klebers auf das Schutzblatt sogar verzichtet werden. Nach dem Zusammenklappen ist der Biosensor dann gebrauchsfertig.

Die Herstellung eines Biosensors mit im Bereich der Knicklinie liegender Aufnahmeöffnung kann besonders bevorzugt dadurch erfolgen, daß im Bereich der Knicklinie ein vorzugsweise linsenförmiges Loch gestanzt wird. Dieses Loch sollte so geformt werden, daß der im Unterteil liegende Rand des Loches nach dem Zusammenklappen über dem im Oberteil liegenden Rand des Loches zu liegen kommt. Es muß also weitgehend symmetrisch sein. Natürlich muß der Schlitz in der Zwischenlage bis zum Loch hin verlaufend ausgebildet werden.

Bei der Stanzung dieses Loches kann die oben erwähnte Ausbildung der Randflächen erfolgen, d. h. die Randfläche des Loches, die im Unterteil liegt, wird senkrecht zur Deckfläche verlaufen und die Randfläche, die im Oberteil liegt, wird schräg verlaufend zur Deckfläche ausgebildet.

Alternativ befindet sich die Auftragsöffnung des Kapillarkanals in einem gestuften Rand. Dann ist das Loch etwas asymmetrisch zu stanzen. Diese Ausführungsform erleichtert dem Patienten ebenfalls den Auftrag eines Bluttropfens. Sie ist einfacher herzustellen, als die mit der schräg verlaufenden Randfläche des Unterteils, bietet aber ähnliche Gebrauchsvorteile.

Der so hergestellte Biosensor ist insbesondere geeignet zur Bestimmung von Blutwerten, insbesondere Blutzucker, Harnstoff, Lactat, Cholesterin, Vitaminen, Troponin und Myoglobin.

Vorteilhafte Weiterbildungen der Erfindung sind Gegenstand der Unteransprüche.

Die Erfindung wird nachfolgend unter Bezugnahme auf die Zeichnungen, deren Offenbarungsgehalt sämtlich erfindungswesentlich ist, in Ausführungsbeispielen näher erläutert. In der Zeichnung zeigt:
Fig. 1 eine Explosionsdarstellung eines Biosensors,
Fig. 2 eine Darstellung einer Trägerplatte eines Biosensors vor dem Zusammenklappen,
Fig. 3 eine Trägerplatte eines Biosensors beim Zusammenklappen und
Fig. 4 eine vergrößerte Schnittdarstellung einer Auftragsöffnung des Biosensors der Figuren 1 bis 3.

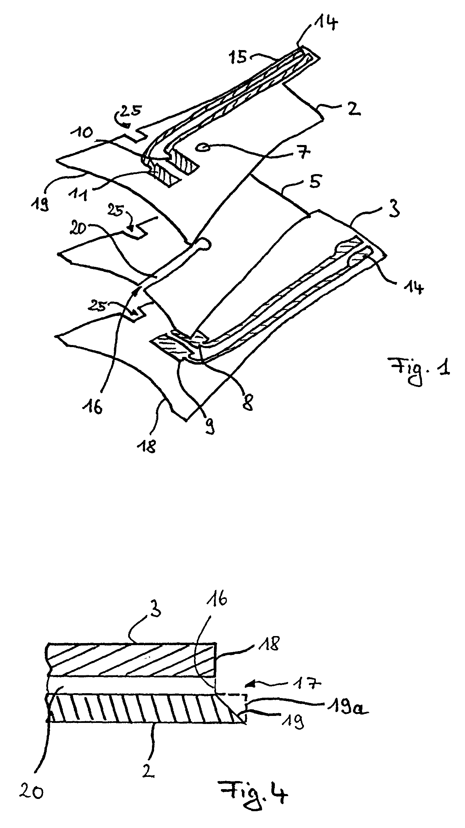

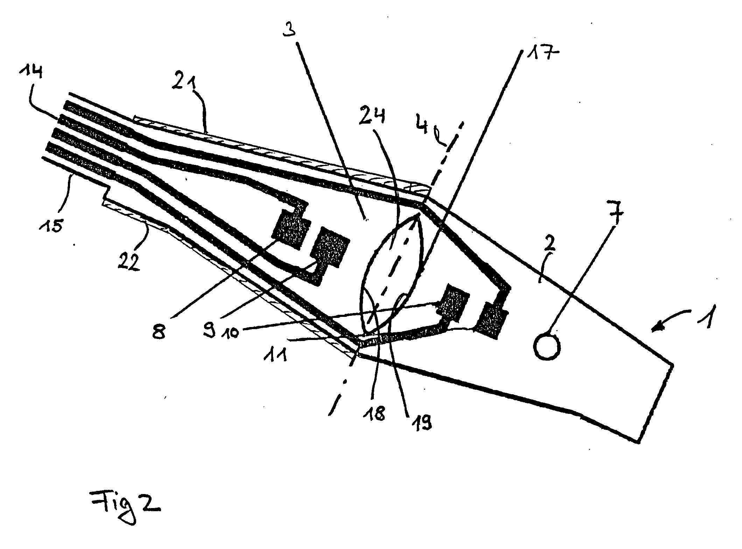

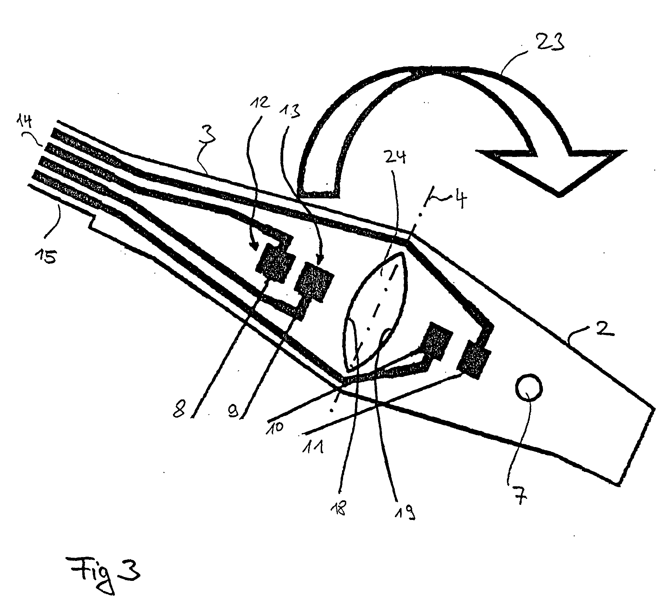

In Figur 1 ist eine Explosionsdarstellung eines Biosensors gezeigt. Dieser Biosensor ist zum Einschieben in ein Auswertegerät ausgebildet. Er weist einen entsprechenden Abschnitt 15 mit Kontakten 14 auf, die nach dem Einschieben in den Schlitz eines Auswertegerätes elektrisch kontaktiert werden. Der Biosensor besteht aus einem Oberteil 2 sowie einem Unterteil 3, die beide aus einer 10µm - 200 µm starken Kunststoff-Folie bestehen. Zwischen Oberteil 2 und Unterteil 3 befindet sich eine Zwischenlage 5, die Ober- und Unterteil verbindet. Im Oberteil 2 ist ein Luftloch 7 gebildet, dessen Funktion später noch erläutert werden wird. Das Oberteil 2 weist an seiner zur Zwischenlage 5 gelegenen Fläche zwei Elektroden 10 und 11 auf, die der besseren Erkennbarkeit halber in Figur 1 allerdings auf der von der Zwischenlage 5 wegweisenden Seite eingezeichnet sind. Das Unterteil 2 weist ähnliche Elektroden 8, 9 auf. Oberteil 2 und Unterteil 3 schließen die Zwischenlage 5 so ein, daß die Elektrode 10 genau der Elektrode 8 und die Elektrode 9 genau der Elektrode 11 gegenüberliegt.

In der Zwischenlage 5 ist ein Schlitz 6 gebildet, der über die Elektrodenpaare 9, 11 und 8, 10 bis zum Luftloch 7 hin verläuft. Der Schlitz 6 beginnt an einem Rand des Biosensors, wie später noch erläutert werden wird. Der Schlitz 6 bildet zusammen mit dem Oberteil 2 und dem Unterteil 3 einen Kapillarkanal 20, der zum Transport von zu analysierender Körperflüssigkeit dient. Er mündet in eine Auftragöffnung 16, die an einem Rand des Biosensors liegt. Die Zwischenlage ist zweilagig aufgebaut. Sie besteht aus einer auf das Unterteil aufgebrachten Lackschicht und einer darauf befindlichen Klebeschicht, die das Oberteil fixiert.

Die Elektroden 8, 9 sind mit Kontakten 14 am Unterteil 3, die Elektroden 10 und 11 mit ebensolchen Kontakten 14 am Oberteil 2 verbunden.

Das Unterteil 3, die Zwischenlage 5 und das Oberteil 2 sind in Passung zusammengefügt, dazu ist in jedem der drei Teile eine Paßstruktur 25 vorgesehen. Beispielsweise kann es sich dabei um Nuten oder Kerben handeln, die miteinander in Deckung gebracht werden. Ist dies erfolgt, sind die Elektroden 11, 9 und 8, 10 der Elektrodenpaare genau gegenüberliegend im Kapillarkanal 20 angeordnet.

Im Bereich der Auftragsöffnung 16 ist der Rand des Biosensors mit einer Einbuchtung 17 versehen, die einen leichten Auftrag der zu analysierenden Körperflüssigkeit, beispielsweise des Blutes aus der Fingerbeere eines Patienten ermöglicht. Die Randflächen 18 und 19 am Unterteil 3 bzw. am Oberteil 2 sind, wie in Figur 4 dargestellt, gestaltet. Figur 4 zeigt einen Teilschnitt durch den Biosensor entlang des Kapillarkanals 20. Die Randfläche 18 der Einbuchtung 17 im Unterteil 2 verläuft senkrecht zur Deckfläche des Unterteils 3. Die Randfläche 19 der Einbuchtung 17 im Oberteil 2 verläuft dagegen schräg zur Deckfläche des Oberteils 2. Dabei liegen die Randflächen 18 und 19 so zueinander, daß die Randfläche 19 über die Kanten des Randes des Unterteils 3 vorspringt. Der Winkel, den die Randfläche 19 zur Deckfläche des Unterteils 3 einnimmt, liegt zwischen 30° und 40°. Diese schräg verlaufende Randfläche dient als Verdrehsicherung und Zentrierung, die den Patienten dabei unterstützt einen beispielsweise auf der Fingerbeere sitzenden Bluttropfen in die Auftragsöffnung 16 einzubringen. Die Randflächen 18 und 19 des Stanzloches 24 sind in Figur 4 dargestellt. Alternativ können die Ränder 18, 19 auch so gestaltet werden, daß sie zwar beide denselben Winkel zur Deckfläche einnehmen, eine Randfläche aber leicht vorspringt, wie dies in Figur 4 gestrichelt als Randfläche 19a gezeigt ist.

In Figur 2 ist eine alternative Ausbildung des Biosensors der Figur 1 dargestellt. In dieser Variante sind die in Figur 1 als getrennte Teile vorgesehenen Ober- bzw. Unterteile 2, 3 an einer Knicklinie 4 miteinander verbunden, d. h. sie sind Bestandteile einer Trägerplatte 1. Diese Trägerplatte 1 ist mit den Elektroden 8 bis 11 sowie den Kontakten 14 und entsprechenden Leiterbahnen bedruckt. Die Zwischenlage wie bei der obigen Ausbildung 5 ist wie bei der obiger Ausbildung gemäß Figur 1 ebenfalls in Drucktechnik auf dem Oberteil 2 oder dem Unterteil 3 aufgebracht. Anschließend wird die Trägerplatte 1 entlang der Knicklinie 4 zusammengeklappt. Falls erforderlich, ist dazu auf der der bedruckten Seite gegenüberliegenden Fläche der Trägerplatte 1 im Bereich der Knicklinie 4 eine entsprechende Nut vorgesehen, um auch bei einer dickeren Trägerplatte 1 ein einfaches Zusammenklappen zu gewährleisten. Wie das Zusammenklappen erfolgt, wird später noch anhand Figur 3 erläutert werden.

Der Kapillarkanal mündet nun in den Rand des Biosensors, an dem die Knicklinie 4 liegt. Die Einbuchtung 17 ist deshalb in der Trägerplatte als linsenförmiges Stanzloch 24 ausgebildet.

Der durch das Oberteil 2, das Unterteil 3 und den Schlitz 6 gebildete Kapillarkanal kann als Schrägkapillare ausgebildet werden. Dazu weitet sich der Kapillarkanal von der Auftragsöffnung 16 zum Luftloch 7 hin auf oder verjüngt sich. Dies bewirkt ein unterschiedliches Fließverhalten. Verjüngt sich der Kapillarkanal von der Auftragsöffnung 16 zum Luftloch 7 hin, so erreicht man ein langsames Fließverhalten der Körperflüssigkeit. Weitet sich der Kapillarkanal 20 von der Auftragsöffnung 16 zum Luftloch 7 hin auf, erhält man ein schnelleres Fließverhalten.

Das Luftloch 7 ist wesentlich, da nur dann die Kapillarkräfte ein ausreichend schnelles Einsaugen einer Körperflüssigkeit in den Kapillarkanal 20 bewirken.

Auf jeweils einer Elektrode 8 oder 11 sowie 9 oder 10 der Elektrodenpaare 8, 11 und 9, 10 ist eine enzymhaltige Substanz aufgebracht. Dabei kann eines der folgenden Enzyme verwendet werden: Lactatoxidase, Glucoseoxidase, Cholesterase, Uricase, Xanthinoxidase, Peroxidase, Urease, Aminotransferase, Cholesterinoxidase, Aminooxidasen, Glutamatoxidase, Kreatininoxidase, Kreatininaminohydrolase und Dehydrogenasen.

Auf das Funktionsprinzip der Messung wird später noch eingegangen werden.

Der Sensor der Figur 2 wird nun folgendermaßen hergestellt. Zuerst wird die Trägerplatte 1 hergestellt, beispielsweise indem sie aus einer größeren Platte ausgestanzt wird. Als Material für die Trägerplatte kommen geeignete inerte Kunststoffe, insbesondere als Folienmaterial, in Frage.

Anschließend werden das Luftloch 7 sowie das Stanzloch 24 gestanzt. Für das Stanzloch 24 wird ein spezielles Stanzverfahren eingesetzt, um sowohl die gerade Randfläche 18 als auch die schräg verlaufende Randfläche 19 in einem Schritt stanzen zu können. Dazu wird ein zweistufiges Messer verwendet, das zuerst mit einem ersten Messerabschnitt das Stanzloch 24 mit geraden Randflächen ausstanzt und anschließend mit einem zweiten Messerabschnitt die schräge Randfläche 19 im Unterteil 2 schafft. Sollt die vereinfachte Form der Verdrehsicherung mit geraden nicht genau fluchtenden Kanten hergestellt werden, muß das Stanzloch 24 asymmetrisch, z. B. leicht verschoben zur Knicklinie 4 liegend gestanzt werden, so daß die Randfläche 19a leicht gegenüber der Randfläche 18 bezogen auf die Symmetrie zur Knicklinie 4 versetzt ist. Auf das spezielle ein zweistufiges Messer verwendende Stanzverfahren kann dann verzichtet werden.

Nach dem Stanzvorgang wird mit einem per se bekannten Siebdruckverfahren eine Leiterstruktur aufgebracht, die aus den Elektroden 8 bis 11, den Kontakten 14 sowie entsprechenden Verbindungsleitungen besteht. Als Material für die Leitungsstruktur bietet sich aufgrund seiner guten Kontakteigenschaften Gold an, es kommen aber auch Graphit, Kupfer, Aluminium, Silber, Platin usw. in Frage.

Das Unterteil 3 hat dabei einen Abschnitt 15 auf dem die Kontakte 14 zu liegen kommen. Dieser Abschnitt 15 ist zum Einstecken in ein Auswertegerät ausgebildet.

Mit einem Rändelverfahren wird anschließend am Abschnitt 15 oder daran anschließend eine Druckprägung an den Rändem der Trägerplatte 1 eingearbeitet, die später als Lichtleitelemente 21 bzw. 22 dient. Wird bei eingestecktem Biosensor vom Auswertegerät Licht in diese Lichtleitelemente 21 oder 22 eingestrahlt, leuchten sie in der Farbe des eingestrahlten Lichtes. Dies dient als Hinweissignal für den Patienten.

Nach dem Aufbringen der Leiterstruktur wird auf dem Unterteil 2 die Zwischenschicht 5 mit einem Siebdruckverfahren aufgebracht. Zuerst wird eine Lackschicht in geeigneter Struktur aufgebracht. Dieser Lack ist 2-5 mm dick und läßt den Bereich des Schlitzes 20 sowie die Elektroden 8 und 9 frei. Er dient als Abstandshalter und haftet direkt auf dem Unterteil 3. Auf den Lack wird eine dünne Klebstoffschicht aufgebracht. Dabei handelt es sich um einen speziellen Klebstoff, der das später noch aufzubringende Enzym nicht schädigt. Die Klebemasse wird beim Druck ebenfalls so strukturiert, daß vom Luftloch 7 zum Stanzloch 24 hin der Schlitz 6 frei bleibt. Man kann die durch die Klebemasse gebildete Zwischenlage 5 auch auf das Oberteil 2 aufbringen.

In dieser Stufe des Verfahrens ist es möglich, den Herstellvorgang nahe zu beliebig lang zu unterbrechen. Man muß lediglich die Klebemasse der Zwischenlage 5 mit einer Schutzfolie abdecken. Dies hat den Vorteil, daß der nachfolgende Schritt, bei dem eine evtl. verderbliche, enzymhaltige Substanz aufgebracht wird, relativ kurz vor dem Endvertrieb des fertigen Sensors erfolgen kann. Die bis dahin vorbereitete Trägerplatte 1 kann so in großen Stückzahlen vorproduziert werden.

Zur Fertigstellung des Biosensors wird, gegebenenfalls nach Abziehen der Schutzfolie, auf die Elektroden 8, 9 des Unterteils 3 oder die Elektroden 10, 11 des Oberteils 2 eine pastöse enzymhaltige Substanz aufgebracht. Dabei ist der Viskositätsgrad diese Substanz so eingestellt, daß die Substanz nicht zwischen den Elektroden verläuft und sich nicht mischt. Durch diese Einstellung des Viskositätsgrades ist es möglich, unterschiedlich zubereitete Pasten auf die zwei Elektroden aufzubringen. Die Pasten haben nach dem Auftrag eine Dicke von 2 bis 11 µm.

Dann wird die Trägerplatte 1 entlang der Knicklinie 4 zusammengeklappt, wobei die als Zwischenlage 5 dienende Klebemasse das Unterteil 2 mit dem Oberteil 3 verklebt. Dieser Zusammenklappvorgang ist durch den Pfeil 23 der Figur 3 symbolisiert, in der allerdings der besseren Erkennbarkeit halber die Zwischenlage 5 nicht mit eingezeichnet ist. Der Biosensor ist dann gebrauchsfertig.

Die Verwendung des Biosensors erfolgt nun folgendermaßen.

Zuerst wird der Biosensor in ein Auswertegerät eingesteckt, das die Kontakte 14 elektronisch anschließt und somit eine Verbindung zu den Elektroden 8 bis 11 herstellt.

Dann muß sich der Patient zur Gewinnung eines Bluttropfens in die Fingerbeere stechen, wenn beispielsweise der Blutzuckerwert bestimmt werden soll. Anschließend legt der Patient den Finger mit dem Bluttropfen in die Einbuchtung 17. Dabei kommt ihm die Zentrierfunktion durch die Randflächengestaltung an der Auftragsöffnung 16 zugute: Er muß den Finger nicht schräg auf die Einbuchtung 17 hinführen sondern die Randfläche, beispielsweise die schräge Randfläche 19 führt den Finger automatisch auf die Auftragsöffnung 16.

Durch die Kapillarkräfte, die je nach Ausgestaltung des Kapillarkanals 20 als Schrägkapillarkanal unterschiedlich stark sind, wird der Bluttropfen durch den Kapillarkanal 20 über die Elektrodenpaare 11, 12 und 10, 8 gezogen.

Zwischen jedem Elektrodenpaar läuft dann folgender Prozeß ab.

In der jeweils auf eine Elektrode des Elektrodenpaares aufgebrachten Paste befindet sich ein substanz-spezifisches Enzym, so daß eine Redox-Reaktion stattfindet, bei der Elektronen von der zu bestimmenden Substanz, beispielsweise Glucose, auf das Enzym unter Umwandlung der zu bestimmenden Substanz in einen Metaboliten übertragen wird. Anschließend werden die Elektroden vom in der Substanz befindlichen Enzym auf eine Elektrode übertragen, gegebenenfalls kann das durch einen bekannten Mediator, z. B. Ferrocen erleichtert werden.

Der so erzeugte Strom wird gemessen. Alternativ zur elektrochemischen Messung kann man auch die Leitfähigkeit erfassen.

Die elektrochemische Änderung wird nun über einen bestimmten Zeitraum kontinuierlich verfolgt. Daraus ergibt sich eine Kurve, die hinsichtlich verschiedener Kenngrößen ausgewertet werden kann, beispielsweise auf ihre Steigung, die absolute Höhe usw. Dies ist dem Fachmann bekannt.

Je nach Resultat des Meßergebnisses zeigt das Auswertegerät dann den Anteil der zu bestimmenden Substanz im Blut an. Dabei kann eine qualitative Anzeige der nachzuweisenden Substanz erfolgen, z. B. im Sinne einer ja/nein-Aussage, ob ein Schwellwert überschritten ist; es ist aber auch eine quantitative Bestimmung, z. B. durch Angabe einer Konzentration möglich. Weiter ist es möglich, bei Überschreitung eines einzustellenden Grenzwertes einen zusätzlichen Hinweis zu geben oder das Meßergebnis auf Plausibilität zu überprüfen.

Der Biosensor erlaubt es nun, aufgrund der mindestens zwei Elektrodenpaare aus einer einzigen Körperflüssigkeitsprobe mehrere Meßwerte zu gewinnen. Dazu sind jeweils unterschiedlich zubereitete Pasten mit unterschiedlichen Enzymen an den Elektrodenpaaren vorzusehen. Alternativ ist es möglich, ein Elektrodenpaar für eine Referenzmessung zu verwenden. Die Verhältnisse der Referenzmessung entsprechen der oben erwähnten Blutzuckermessung mit dem Unterschied, daß die verwendete Substanz nicht enzymhaltig ist, ansonsten aber möglichst dieselben Eigenschaften aufweist. Die Referenzmessung kann dann ebenfalls mit bestimmten Grenzwerten verglichen werden, z. B. um eine Plausibilitätsüberprüfung durchzuführen. Weiter wird die Referenzmessung nach den selben Kriterien ausgewertet, wie die Messung an dem Elektrodenpaar mit enzymhaltiger Paste, wobei die Meßergebnisse der Referenzmessung als Nullwert dienen. Damit kann die Meßgenauigkeit gesteigert werden, wie es beispielsweise auch aus der erwähnten EP-A 0 359 831 bekannt ist.

Beim Durchführen der Messung dienen die Lichtleitelemente 21, 22 dazu, dem Patienten Informationen zu vermitteln. Beispielsweise kann das Lichtleitelement 21 im Auswertegerät an eine rote LED und das Lichtleitbündel 21 an eine grüne LED angekoppelt sein. Damit kann man dem Patienten anzeigen, ob der Biosensor zu einer Messung bereit ist, nachdem er in das Auswertegerät eingesteckt wurde. Beispielsweise kann man die an die grüne LED angekoppelte Lichtleitfläche 21 in dem Zeitraum erleuchten, in dem der Patient die Körperflüssigkeit in die Auftragsöffnung 16 einbringen kann. Wurde vom Auswertegerät erkannt, daß eine ausreichende Menge an Körperflüssigkeit aufgebracht wurde, kann die Beleuchtung der Lichtleitelemente 21, 22 entsprechend umgeschaltet werden, um den Patienten, beispielsweise durch ein rotes Licht zu signalisieren, daß eine gültige Messung erfolgte bzw. das Sensorelement nicht für die Aufnahme weiterer Körperflüssigkeiten bereit ist.

## Patentansprüche

1. Biosensor zum Bestimmen von Substanzen in Körperflüssigkeiten, insbesondere in Blut, mit
- einem Oberteil (2) und einem Unterteil (3), die aufeinander liegen, und
- einer zwischen Oberteil (2) und Unterteil (3) liegenden Zwischenlage (5) in der ein Schlitz (6) gebildet ist, wobei
- Oberteil (2), Unterteil (3) und Schlitz (6) einen Kapillarkanal (20) bilden, der von einer am Rand des Biosensors gebildeten Auftragsöffnung (16) zu einem im Ober- oder Unterteil (2, 3) gebildeten Luftloch (7) verläuft, und
- Elektroden vorgesehen sind, die zusammen mit einer enzymhaltigen Substanz eine elektrochemische Messung der zu bestimmenden Substanzen erlauben, **dadurch gekennzeichnet, daß**
- sowohl das Oberteil (2) als auch das Unterteil (3) im Bereich des Kapillarkanals (20) jeweils mindestens eine Elektrode (8, 9; 10, 11) trägt, wobei die Elektroden im Kapillarkanal (20) paarweise gegenüberliegen und auf mindestens einer Elektrode (8, 9) mindestens eines Elektrodenpaars eine enzymhaltige Substanz (12, 13) aufgebracht ist.

2. Biosensor nach Anspruch 1, **dadurch gekennzeichnet, daß** Ober- und Unterteil (2, 3) Teile einer Trägerplatte (1) sind, die entlang einer Knicklinie (4) zusammengeklappt ist, an der Ober- und Unterteil (2, 3) miteinander verbunden sind.

3. Biosensor nach Anspruch 2, **dadurch gekennzeichnet, daß** die Elektroden (8, 9; 10, 11) über Leiterstreifen mit Kontakten (14) verbunden sind, die am Ober- und/oder Unterteil (2, 3) an einem, vorzugsweise über das Unter- oder Oberteil (3, 2) überstehenden Abschnitt (15) liegen, der zum Einstecken in ein Auswertegerät ausgebildet ist.

4. Biosensor nach Anspruch 2 oder 3, **dadurch gekennzeichnet, daß** die Auftragsöffnung (16) am von der Knicklinie (4) gebildeten Rand des Biosensors liegt.

5. Biosensor nach einem der vorhergehenden Ansprüche, **dadurch gekennzeichnet, daß** die Auftragsöffnung (16) als bogenförmige Einbuchtung (17) im Ober- und im Unterteil (2, 3) gestaltet ist.

6. Biosensor nach Anspruch 5, **dadurch gekennzeichnet, daß** die Einbuchtung (17) im Unterteil (3) eine bezogen auf die Deckfläche des Unterteils (3) senkrecht liegende Randfläche (18) und im Oberteil (2) eine bezogen auf die Deckfläche des Oberteils (3) geneigt liegende Randfläche (19) hat.

7. Biosensor nach Anspruch 3, **dadurch gekennzeichnet, daß** die Breite des Schlitzes (6) in der Zwischenlage (5) von der Auftragsöffnung (16) weg gesehen zu- oder abnimmt, so daß der Kapillarkanal 20 als Schrägkapillarkanal ausgebildet ist.

8. Biosensor nach Anspruch 3, **dadurch gekennzeichnet, daß** an einem Rand oder an beiden Rändern des den überstehenden Abschnitt (15) aufweisenden Ober- oder Unterteils (2, 3) ein Lichtleitelement (21, 22) gebildet ist.

9. Biosensor nach einem der vorhergehenden Ansprüche, **dadurch gekennzeichnet, daß** das Enzym in der enzymhaltigen Substanz von der Gruppe Lactatoxidase, Glucoseoxidase, Cholesterase, Uricase, Xanthinoxidase, Peroxidase, Urease, Aminotransferase, Cholesterinoxidase, Aminooxidase, Glutamatoxidase, Kreatininoxidase, Kreatininaminohydrolase und Dehydrogenase ausgewählt ist.

10. Verfahren zum Herstellen eines Biosensors gemäß einem der vorhergehenden Ansprüche mit folgenden Schritten:
a) Herstellen einer Trägerplatte, die zwei verbundene Teile aufweist, welche um eine Knicklinie zusammenklappbar sind, wobei das eine Teil der Trägerplatte ein Oberteil und das andere Teil ein Unterteil des Biosensors darstellt,
b) Bilden eines Durchtrittsloches in der Trägerplatte,
c) Aufbringen einer Leiterstruktur aufweisend Elektroden, die durch Leiterbahnen mit Kontakten auf dem Ober- oder dem Unterteil verbunden sind, wobei mindestens eine Elektrode auf dem Oberteil und ebenso viele Elektroden auf dem Unterteil sitzen, und
d) Aufbringen einer Zwischenlage auf das Ober- oder das Unterteil, wobei in der Zwischenlage ein Schlitz vorgesehen wird, der zum Loch läuft und über den Elektroden des Ober- oder Unterteils liegt.

11. Verfahren nach Anspruch 10, **dadurch gekennzeichnet, daß** nach Schritt d) folgender Schritt durchgeführt wird:
e) Aufbringen einer enzymhaltigen Paste auf jeder im Schlitz liegenden Elektrode.

12. Verfahren nach Anspruch 11, **dadurch gekennzeichnet, daß** nach Schritt e) die Trägerplatte entlang der Knicklinie zusammengeklappt wird, so daß die Zwischenlage zwischen Oberteil und Unterteil zu liegen kommt, und daß aus dem Schlitz, dem Ober- und dem Unterteil ein Kapillarkanal gebildet wird, in dem die Elektroden auf dem Ober- und dem Unterteil paarweise gegenüberliegen.

13. Verfahren nach einem der vorhergehenden Verfahrensansprüche, **dadurch gekennzeichnet, daß** vorzugsweise vor Schritt c) in die Trägerplatte im Bereich der Knicklinie ein vorzugsweise linsenförmiges Loch gestanzt wird, daß so geformt ist, daß der im Unterteil liegende Rand des Loches beim Zusammenklappen im Bereich des im Oberteil liegenden Randes des Loches zu liegen kommt, und daß der Schlitz zum Loch hin verlaufend ausgebildet wird.

14. Verfahren nach Anspruch 13, **dadurch gekennzeichnet, daß** bei der Stanzung des Loches der im Unterteil liegende Rand des Loches eine senkrechte Randfläche und der im Oberteil liegende Rand des Loches eine schräge Randfläche erhält und daß beim Zusammenklappen der im Unterteil liegende Rand des Loches beim Zusamenklappen auf dem im Oberteil liegenden Rand des Loches zu liegen kommt.

15. Verwendung des Biosensors nach einem der vorhergehenden Ansprüche 1 bis 9 zum Bestimmen mindestens eines Blutwertes, vorzugsweise aus der Gruppe: Blutzuckerwert, Harnstoff, Lactat, Cholesterin, Vitaminen, Troponin und Myoglobin.
